# EUROPEAN PATENT SPECIFICATION

(11) **EP 3 099 357 B1**
(45) Date of publication and mention of the grant of the patent: **30.12.2020**
(21) Application number: 15702707.9
(22) Date of filing: 27.01.2015
(51) Int. Cl.: A61M 5/32

(54) **MEDICAMENT DELIVERY DEVICE**
MEDIKAMENTENABGABEVORRICHTUNG
DISPOSITIFS D'ADMINISTRATION DE MÉDICAMENTS

(30) Priority: 30.01.2014 EP 14153203
(43) Date of publication of application: 07.12.2016
(73) Proprietor: Sanofi-Aventis Deutschland GmbH, 65929 Frankfurt am Main (DE)
(72) Inventor: HUTHMACHER, Winfried, 60389 Frankfurt (DE)
(74) Representative: Finger, Catrin
(86) International application number: PCT/EP2015/051595
(87) International publication number: WO 2015/113969

(56) References cited:
- WO-A1-2007/020090
- WO-A1-2007/047200
- WO-A1-2012/000833
- WO-A1-2012/000837
- WO-A1-2012/093072

## Description

### Technical Field

The invention relates to a medicament delivery device.

### Background of the Invention

Administering an injection is a process which presents a number of risks and challenges for users and healthcare professionals, both mental and physical. Pre-filled syringes that are filled with a selected dosage of a medicament for administering the medicament to a patient are known in the art. Medicament delivery devices comprising a needle sleeve for covering a needle of a pre-filled syringe before and after use are also known. Typically, the needle sleeve is either manually moved or moved by the action of a relaxing spring to surround the needle.

WO 2012/000833 A1 discloses a safety device for a pre-filled syringe comprising a hollow support body having a releasable mounting means adapted to mount a pre-filled syringe within the support body, a hollow outer body sildably arranged with respect to the support body and adapted to receive the support body and a release collar non-rotatably arranged within the support body. The release collar is movable relative to the support body and adapted to release the mounting means. A guide track is formed into at least one substantially planar side wall of the support body. The release collar comprises at least one flexible arm with a guide pin that extends from the flexible arm and protrudes through the guide track. The guide pin is movable within and along the guide track in a plane defined by the substantially planar side wall. A guide rail is formed into at least one substantially planar inner surface of the outer body. The guide rail is adapted to abut against the guide pin to guide the movement of the guide pin along the guide track when the outer body is slid relative to the support body.

WO 2012/093072 A1 discloses a safety device for a pre-filled syringe with an injection needle comprising a support body adapted to mount the pre-filled syringe, a needle shield slidably arranged with respect to the support body, a retention and locking means for retaining and locking the needle shield with respect to the support body in a first and a second advanced position and in a retracted position. The needle shield comprises an annular flange adapted to rest on the skin of the patient receiving an injection. The retention and locking means are arranged at a distal end of the safety device.

WO 2012/000837 A1 discloses an injection device comprising a pre-filled syringe and a safety device. The pre-filled syringe comprises a barrel with an inner cavity in fluid communication with a hypodermic needle attached to a distal end of the pre-filled syringe and a piston fluid-tightly sealing a proximal end of the inner cavity. The safety device comprises a support body for retaining the pre-filled syringe therein, an outer body operably connected to the piston, a needle shield and a releasable retaining means. The retaining means comprises a radial projection and a longitudinal recess adapted to receive the radial projection. The radial projection is connected to or integral part of one of the support body or the outer body and a longitudinal recess formed into the other of the support body or the outer body. The needle shield, the support body and the outer body are telescopable relative to each other. The piston is movable in a distal direction to expel a medicament contained in the inner cavity through the hypodermic needle by a manual actuation of the outer body. A telescoping movement of the outer body with respect to the support body is restricted by the retaining means when the needle shield is moved with respect to the support body in a proximal direction from an initial position towards a retracted position. The retaining means is released when the needle shield reaches the retracted position allowing for a telescoping movement of the outer body with respect to the support body to expel the medicament contained in the pre-filled syringe through the hypodermic needle.

WO 2007/047200 A1 discloses a pharmaceutical delivery apparatus including a housing, a syringe assembly, and a needle cap. The syringe assembly is plungeable relative to the housing from a first position, at which its needle tip is disposed within the housing, to a second position, at which its needle tip projects from the housing beyond the proximal end for insertion into an injection site. A base of the needle cap is exposed at the housing proximal end to be manually grippable for cap removal. A needle cap stem is upstanding from the base and sized and configured to insert through an opening is the housing proximal end to cover the needle tip when the syringe assembly is disposed in the first position. The needle cap base further includes a plurality of distally projecting cams located radially outward of the stem. The cams are fittable within slots in the housing proximal end when the cap is fully mounted to the apparatus.

There remains a need for an improved medicament delivery device.

### Summary of the Invention

It is an object of the present invention to provide an improved medicament delivery device.

The medicament delivery device according to the present invention is defined in claim 1. It comprises a piston rod and a container carrier for retaining a medicament container within the body. At least one longitudinal rib associated with the piston rod is arranged to engage the carrier. At least one of the carrier and the at least one rib comprises a resilient material adapted to be deformed by the engagement between the carrier and the at least one rib.

The medicament delivery device further comprises a body. The container carrier is arranged within the body. The piston rod is connected to the body, and the at least one rib is arranged within the body in a manner to protrude in a radial inward direction. The carrier is movable from an initial position in a proximal direction relative to the body.

The medicament delivery device further comprises a sleeve movable within the body along a longitudinal axis so as to cover or expose an injection needle. In an exemplary embodiment, the container carrier comprises a transverse portion adapted to support a flange on a syringe barrel of a syringe such that the syringe is prevented from moving in a distal direction relative to the carrier. The transverse portion is attached to a ring shaped collar adapted to engage the at least one rib. The collar has a substantially circular cross section when relaxed, and an internal diameter of the collar is greater than a maximum external diameter of the flange of the syringe. The transverse portion comprises an inner collar connected to first sections of the collar by a number of connecting ribs. The inner collar has an internal diameter substantially corresponding to an external diameter of the syringe barrel. On axial movement of the collar relative to the inner collar the connecting ribs are adapted to tilt the first sections with respect to the longitudinal axis. One or more carrier ribs are arranged on an inner surface of the collar and protrude in a radial inward direction in a manner to engage the flange on the syringe barrel for restricting movement of the syringe in the proximal direction relative to the carrier. The carrier rib comprises a proximal ramp.

The rib comprises a step adapted to allow the carrier being moved until proximally abutting the step without being deformed or with a lower extent of deformation and adapted to deform the carrier to a greater extent proximally from the step. A sleeve spring is arranged between the carrier and the sleeve in a manner biasing the sleeve (6) in the distal direction relative to the carrier.

Further scope of applicability of the present invention will become apparent from the detailed description given hereinafter. However, it should be understood that the detailed description and specific examples, while indicating exemplary embodiments of the invention, are given by way only. of illustration

### Brief Description of the Drawings

The present invention will become more fully understood from the detailed description given hereinbelow and the accompanying drawings which are given by way of illustration only, and thus, are not limitive of the present invention, and wherein:
- Figure 1A: is a schematic longitudinal section of an exemplary embodiment of a medicament delivery device according to the present invention before use,
- Figure 1B: is a schematic perspective view of an exemplary embodiment of a container carrier according to the present invention,
- Figure 2: is a schematic view of an exemplary embodiment of a medicament delivery device according to the present invention during use,
- Figure 3: is a schematic cross sectional view of an exemplary embodiment of a medicament delivery device according to the present invention,
- Figure 4: is a perspective detail view of an exemplary embodiment of a medicament delivery device according to the present invention during use,
- Figure 5: is a schematic cross sectional view of an exemplary embodiment of a medicament delivery device according to the present invention,
- Figure 6: is a schematic view of an exemplary embodiment of two flexible ribs engaged to a pin, and
- Figure 7: is a schematic view of an exemplary embodiment of a flexible rib engaged to a plurality of pins.

Corresponding parts are marked with the same reference symbols in all figures.

### Detailed Description

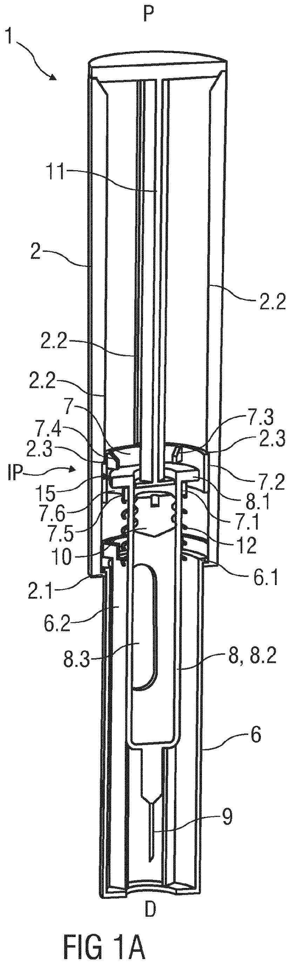

Figure 1A is a schematic longitudinal section of an exemplary embodiment of a medicament delivery device 1 according to the present invention. The medicament delivery device 1 comprises a substantially elongate and cylindrical body 2.

The medicament delivery device 1 furthermore comprises a sleeve 6 with a proximal collar 6.1 and one or more longitudinal ribs 6.2 adapted to center a medicament container within the sleeve 6. The sleeve 6 is slidably coupled to the body 2 for allowing relative movement along a longitudinal axis in a distal direction D and/or a proximal direction P.

A container carrier 7 for retaining a medicament container (e.g., a syringe 8) is arranged within the body 2.

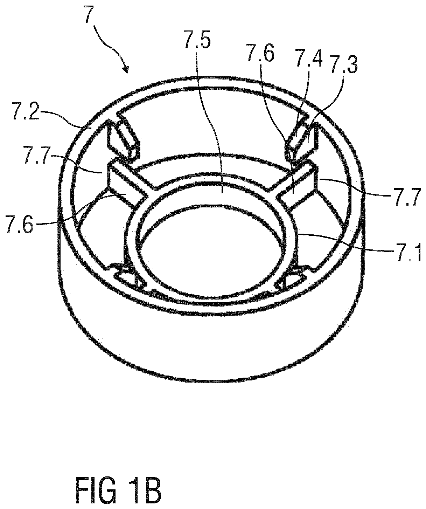

Figure 1B is a schematic perspective view of an exemplary embodiment of a container carrier 7.

In an exemplary embodiment, the carrier 7 comprises a resilient material, e.g. a plastic, allowing the carrier 7 to be deformed. In an exemplary embodiment, the container carrier 7 comprises a transverse portion 7.1 adapted to support a flange 8.1 on a syringe barrel 8.2 of the syringe 8 such that the syringe 8 is prevented from moving in the distal direction D relative to the carrier 7. The carrier 7 is slidably disposed within the body 2. The carrier 7 further comprises a collar 7.2 coupled to the transverse portion 7.1. One or more carrier ribs 7.3 are arranged on an inner radial surface of the collar 7.2 and protrude in a radial inward direction in a manner to engage the flange 8.1 on the syringe barrel 8.2 for restricting movement of the syringe 8 in the proximal direction P relative to the carrier 7. The carrier ribs 7.3 may be uniformly distributed over the circumference of the collar 7.2. For example, four carrier ribs 7.3 may be arranged angularly spaced from each other by 90 degrees, respectively. Different numbers of carrier ribs 7.3 with a uniform or non-uniform distribution may likewise be arranged. The collar 7.2 may have a substantially circular cross section with an internal diameter greater than a maximum external diameter of the flange 8.1 of the syringe 8, i.e. a substantially ring shaped clearance 15 is provided between the collar 7.2 and the flange 8.1 allowing for a certain extent of deformation of the collar 7.2.

In an exemplary embodiment, the carrier ribs 7.3 comprise proximal ramps 7.4 adapted to facilitate radial outward deflection of the carrier ribs 7.3 by the flange 8.1 when assembling a syringe 8 to the carrier 7.

In the illustrated embodiment, the transverse portion 7.1 comprises an inner collar 7.5 connected to first sections 7.7 of the collar 7.2 by a number of connecting ribs 7.6. The first sections 7.7 are separated by second sections 7.8 which are not connected to connecting ribs 7.6. In an exemplary embodiment the connecting ribs 7.6 are angularly aligned with the carrier ribs 7.3. The inner collar 7.5 has an internal diameter substantially corresponding to an external diameter of the syringe barrel 8.2. In another exemplary embodiment, the transverse portion 7.1 could be arranged as a transverse disk connected to the collar 7.2. Likewise, the connecting ribs 7.6 may not be aligned with the carrier ribs 7.3 or the numbers of connecting ribs 7.6 may be different from the number of carrier ribs 7.3.

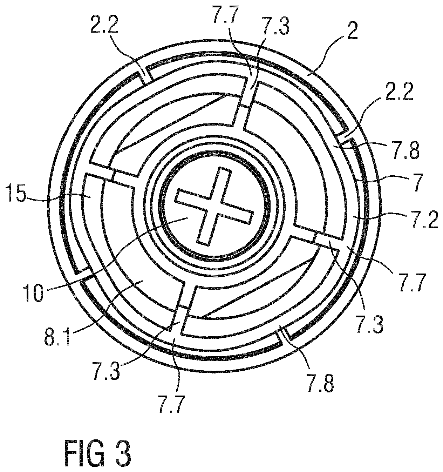

Referring again to figure 1A the body 2 comprises one or more longitudinal body ribs 2.2 adapted to radially outwardly abut the collar 7.2 of the carrier 7. When the body ribs 2.2 abut the collar 7.2, the collar 7.2 may be deformed by the body ribs 2.2 such that the collar's 7.2 cross section is no longer circular (as shown in Figure 3). In an exemplary embodiment, the number of the body ribs 2.2 equals the number of carrier ribs 7.3 and/or connecting ribs 7.6, wherein the carrier 7 is arranged within the body 2 such that the carrier ribs 7.3 and/or connecting ribs 7.6 are angularly offset from the body ribs 2.2. In an exemplary embodiment the carrier ribs 7.3 and/or connecting ribs 7.6 may be equally spaced from the neighbouring body ribs 2.2 (cf. fig. 3). For example, the carrier 7 may be splined to the body 2 in such a manner that the carrier 7 is in a defined angular position relative to the body 2. The body ribs 2.2 respectively comprise a step 2.3, wherein the internal diameter defined by the body ribs 2.2 is greater distally from the steps 2.3 than proximally. The carrier 7 can be moved until proximally abutting this step 2.3 without being deformed or with only a small extent of deformation. In order to move proximally beyond the step 2.3 the collar 7.2 of the carrier 7 must be deformed more. The force required for deforming the collar 7.2 is defined by the resilience of the collar 7.2 and by the geometry of the collar 7.2 and the body ribs 2.2.

Referring again to figure 1A, the syringe barrel 8.2 is arranged as a hollow cylinder defining a cavity 8.3 for receiving a drug. A hollow injection needle 9 is arranged at a distal end of the syringe barrel 8.2 in a manner to be in fluid communication with the cavity 8.3. A stopper 10 is disposed within the syringe barrel 8.2 for proximally limiting the cavity 8.3. The stopper 10 may be displaced within the syringe barrel 8.2 for ejecting the drug from the cavity through the needle 9.

A piston rod 11 is arranged within the body 2 in a manner to engage the stopper 10 for displacing it within the syringe barrel 8.2. In an exemplary embodiment the piston rod 11 is attached to the body 2 preventing relative movement between the piston rod 11 and the body 2. In the illustrated embodiment the piston rod 11 is integrally shaped with the body 2. In another exemplary embodiment the piston rod 11 may be secured to the body 2, e.g., by latches.

A sleeve spring 12 is arranged between the carrier 7 and the longitudinal ribs 6.2 in the sleeve 6 in a manner biasing the sleeve 6 in the distal direction D relative to the carrier 7 such that the collar 6.1 of the sleeve 6 abuts an axial stop 2.1 in the body 2, whereas the collar 7.2 of the carrier 7 abuts the step 2.3 in the body 2. The carrier 7 is hence in an initial position IP relative to the body 2.

In order to perform an injection, the medicament delivery device 1 may be operated according to the following exemplary method.

If applicable, a protective needle sheath is removed from the needle 9.

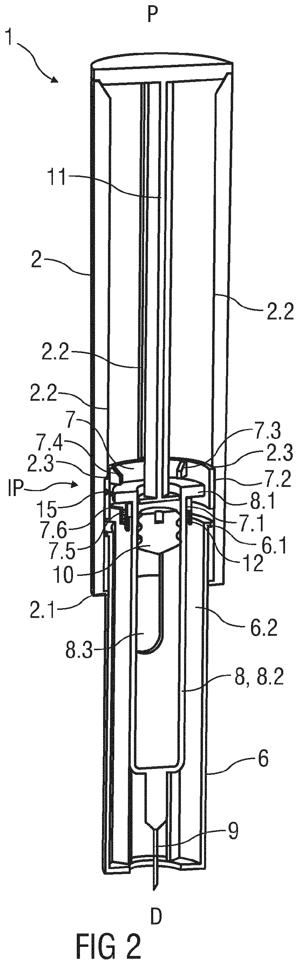

The needle 9 is located within the sleeve 6 preventing a user from touching and seeing it. In this state the medicament delivery device 1 may be held at the body 2 and the sleeve 6 may be pushed against an injection site, e.g. a patient's skin. Consequently the sleeve 6 moves in the proximal direction P relative to the body 2 against the force of the sleeve spring 12. Figure 2 is a schematic view of the medicament delivery device 1 with the sleeve 6 moved in the proximal direction P within the body 2 and the carrier 7 in the initial position. Figure 3 is a corresponding cross sectional view. Due to the movement of the sleeve 6 in the proximal direction P, the sleeve spring 12 is compressed between the sleeve 6 and the carrier 7. The carrier 7 remains in position as it is stopped by the step 2.3 in the body 2. The resistance of the material of the carrier 7 opposing deformation for disengaging the step 2.3 provides the counterforce against the force of the sleeve spring 12 to hold the carrier 7 in position.

Consequently, the syringe 8 and the needle 9 stay in position relative to the body 2 while the sleeve 6 moves in the proximal direction P and the needle 9 is hence exposed and inserted into the injection site. Once the sleeve spring 12 is fully compressed or if the sleeve 6 abuts the carrier 7, movement of the sleeve 6 relative to the carrier 7, syringe 8 and needle 9 stops. The needle 9 has reached its insertion depth. Any further movement of the sleeve 6 relative to the body 2 in the proximal direction P hence increases the force on the carrier 7 deforming it in such a manner that the collar 7.2 overcomes the step 2.3 and starts moving with the syringe 8 out of the initial position IP relative to the body 2.

The deformation of the carrier 7 may be achieved as follows:
The inner collar 7.5 is moved in the proximal direction P by the sleeve 6 and the at least nearly completely compressed sleeve spring 12 which acts between the sleeve 6 and the inner collar 7.5. At the same time, the collar 7.2 is still abutted against the step 2.3 and does not move. The connecting ribs 7.6 attached on first sections 7.7 on the distal edge of the collar 7.2 pull the distal edge of the first sections 7.7 inwards thereby tilting the first sections 7.7 such that their proximal edges deflect radially outwards. As the overall circumference of the collar 7.2 remains substantially constant this tilting causes a tilting in an opposite direction in second sections 7.8 of the collar 7.2 between the first sections 7.7 which consequently disengage the step 2.3 allowing the collar 7.2 to move along the ribs 2.2.

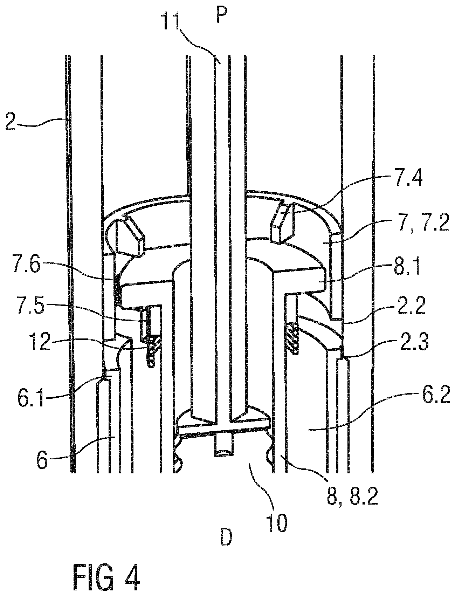

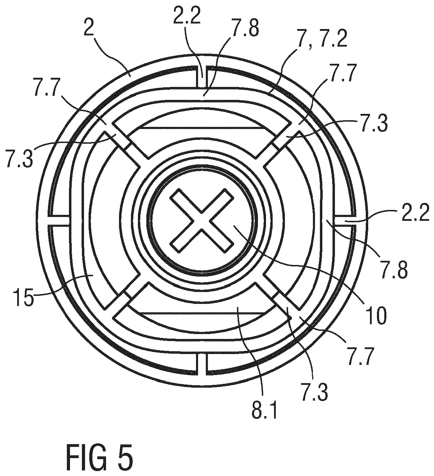

Figure 4 is a perspective detail view of the medicament delivery device 1 with the carrier 7 moved proximally beyond the initial position IP. Figure 5 is a corresponding cross sectional view.

As the piston rod 11 is coupled to the body 2 the movement of the carrier 7 relative to the body 2 causes the piston rod 11 to abut the stopper 10 and displace it within the syringe barrel 8.2 ejecting the drug from the cavity 8.3 through the injection needle 9 into the injection site.

As the carrier 7 moves within the body 2 the controlled distortion of the collar 7.2 by the body ribs 2.2 provides an additional force opposing the delivery of the drug from the cavity 8.3. This force can thus be adjusted by the resilience of the carrier 7 and by the geometry of the body ribs 2.2 and the carrier 7.

In the illustrated embodiments the resilient carrier 7 is applied in a medicament delivery device 1 with manual needle insertion and manual drug delivery. Likewise, the resilient carrier 7 could be applied in an automatic medicament delivery device with automatic needle insertion and/or automatic drug delivery in order to adjust the injection force. In this case the resilient carrier 7 could be arranged to interact with the piston rod 11 for opposing the injection force, wherein the piston rod 11 would not necessarily have to be attached to the body 2.

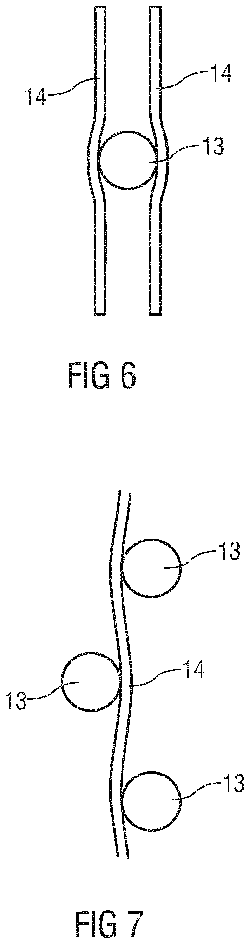

Figure 6 is a schematic view of an exemplary embodiment of an application of flexible material to oppose and control a relative movement between two components. A pin 13 on one of the components, e.g. the body 2 or the carrier 7, may be arranged between two resilient ribs 14 on the other one of the components. The distance between the ribs 14 in the relaxed state is smaller than the diameter of the pin 13. If the pin 13 is moved relative to the ribs 14 it has to overcome the resilience of the ribs 14 and splay them apart.

Figure 7 is a schematic view of an exemplary embodiment of an application of flexible material to oppose a relative movement between two components. A resilient rib 14 on one of the components, e.g. the body 2 or the carrier 7, may be arranged between successive pins 13 on the other one of the components such that the rib 14 is staggered by the pins 13. If the pins 13 are moved relative to the rib they have to overcome the resilience of the rib 14 and change its deformation.

In an exemplary embodiment a cap may be arrangeable over a distal end of the medicament delivery device 1. In an exemplary embodiment a viewing window may be arranged in the cap allowing viewing a syringe arrangeable within the medicament delivery device 1.

The sleeve 6 may comprise one or more lateral sleeve windows adapted to axially align with the viewing windows in the cap in an initial state thus allowing inspection of the syringe contents.

The term "drug" or "medicament", as used herein, means a pharmaceutical formulation containing at least one pharmaceutically active compound,
wherein in one embodiment the pharmaceutically active compound has a molecular weight up to 1500 Da and/or is a peptide, a proteine, a polysaccharide, a vaccine, a DNA, a RNA, an enzyme, an antibody or a fragment thereof, a hormone or an oligonucleotide, or a mixture of the above-mentioned pharmaceutically active compound,
wherein in a further embodiment the pharmaceutically active compound is useful for the treatment and/or prophylaxis of diabetes mellitus or complications associated with diabetes mellitus such as diabetic retinopathy, thromboembolism disorders such as deep vein or pulmonary thromboembolism, acute coronary syndrome (ACS), angina, myocardial infarction, cancer, macular degeneration, inflammation, hay fever, atherosclerosis and/or rheumatoid arthritis,
wherein in a further embodiment the pharmaceutically active compound comprises at least one peptide for the treatment and/or prophylaxis of diabetes mellitus or complications associated with diabetes mellitus such as diabetic retinopathy,
wherein in a further embodiment the pharmaceutically active compound comprises at least one human insulin or a human insulin analogue or derivative, glucagon-like peptide (GLP-1) or an analogue or derivative thereof, or exendin-3 or exendin-4 or an analogue or derivative of exendin-3 or exendin-4.

Insulin analogues are for example Gly(A21), Arg(B31), Arg(B32) human insulin; Lys(B3), Glu(B29) human insulin; Lys(B28), Pro(B29) human insulin; Asp(B28) human insulin; human insulin, wherein proline in position B28 is replaced by Asp, Lys, Leu, Val or Ala and wherein in position B29 Lys may be replaced by Pro; Ala(B26) human insulin; Des(B28-B30) human insulin; Des(B27) human insulin and Des(B30) human insulin.

Insulin derivates are for example B29-N-myristoyl-des(B30) human insulin; B29-N-palmitoyl-des(B30) human insulin; B29-N-myristoyl human insulin; B29-N-palmitoyl human insulin; B28-N-myristoyl LysB28ProB29 human insulin; B28-N-palmitoyl-LysB28ProB29 human insulin; B30-N-myristoyl-ThrB29LysB30 human insulin; B30-N-palmitoyl- ThrB29LysB30 human insulin; B29-N-(N-palmitoyl-Y-glutamyl)-des(B30) human insulin; B29-N-(N-lithocholyl-Y-glutamyl)-des(B30) human insulin; B29-N-(ω-carboxyheptadecanoyl)-des(B30) human insulin and B29-N-(ω-carboxyheptadecanoyl) human insulin.

Exendin-4 for example means Exendin-4(1-39), a peptide of the sequence H-His-Gly-Glu-Gly-Thr-Phe-Thr-Ser-Asp-Leu-Ser-Lys-Gln-Met-Glu-Glu-Glu-Ala-Val-Arg-Leu-Phe-Ile-Glu-Trp-Leu-Lys-Asn-Gly-Gly-Pro-Ser-Ser-Gly-Ala-Pro-Pro-Pro-Ser-NH2.

Exendin-4 derivatives are for example selected from the following list of compounds:
H-(Lys)4-des Pro36, des Pro37 Exendin-4(1-39)-NH2,
H-(Lys)5-des Pro36, des Pro37 Exendin-4(1-39)-NH2,
des Pro36 Exendin-4(1-39),
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39); or
des Pro36 [Asp28] Exendin-4(1-39),
des Pro36 [IsoAsp28] Exendin-4(1-39),
des Pro36 [Met(O)14, Asp28] Exendin-4(1-39),
des Pro36 [Met(O)14, IsoAsp28] Exendin-4(1-39),
des Pro36 [Trp(O2)25, Asp28] Exendin-4(1-39),
des Pro36 [Trp(O2)25, IsoAsp28] Exendin-4(1-39),
des Pro36 [Met(O)14 Trp(O2)25, Asp28] Exendin-4(1-39),
des Pro36 [Met(O)14 Trp(O2)25, IsoAsp28] Exendin-4(1-39),
wherein the group -Lys6-NH2 may be bound to the C-terminus of the Exendin-4 derivative;
or an Exendin-4 derivative of the sequence
des Pro36 Exendin-4(1-39)-Lys6-NH2 (AVE0010),
H-(Lys)6-des Pro36 [Asp28] Exendin-4(1-39)-Lys6-NH2,
des Asp28 Pro36, Pro37, Pro38Exendin-4(1-39)-NH2,
H-(Lys)6-des Pro36, Pro38 [Asp28] Exendin-4(1-39)-NH2,
H-Asn-(Glu)5des Pro36, Pro37, Pro38 [Asp28] Exendin-4(1-39)-NH2,
des Pro36, Pro37, Pro38 [Asp28] Exendin-4(1-39)-(Lys)6-NH2,
H-(Lys)6-des Pro36, Pro37, Pro38 [Asp28] Exendin-4(1-39)-(Lys)6-NH2,
H-Asn-(Glu)5-des Pro36, Pro37, Pro38 [Asp28] Exendin-4(1-39)-(Lys)6-NH2,
H-(Lys)6-des Pro36 [Trp(O2)25, Asp28] Exendin-4(1-39)-Lys6-NH2,
H-des Asp28 Pro36, Pro37, Pro38 [Trp(O2)25] Exendin-4(1-39)-NH2,
H-(Lys)6-des Pro36, Pro37, Pro38 [Trp(O2)25, Asp28] Exendin-4(1-39)-NH2,
H-Asn-(Glu)5-des Pro36, Pro37, Pro38 [Trp(O2)25, Asp28] Exendin-4(1-39)-NH2,
des Pro36, Pro37, Pro38 [Trp(O2)25, Asp28] Exendin-4(1-39)-(Lys)6-NH2,
H-(Lys)6-des Pro36, Pro37, Pro38 [Trp(O2)25, Asp28] Exendin-4(1-39)-(Lys)6-NH2,
H-Asn-(Glu)5-des Pro36, Pro37, Pro38 [Trp(O2)25, Asp28] Exendin-4(1-39)-(Lys)6-NH2,
H-(Lys)6-des Pro36 [Met(O)14, Asp28] Exendin-4(1-39)-Lys6-NH2,
des Met(O)14 Asp28 Pro36, Pro37, Pro38 Exendin-4(1-39)-NH2,
H-(Lys)6-desPro36, Pro37, Pro38 [Met(O)14, Asp28] Exendin-4(1-39)-NH2,
H-Asn-(Glu)5-des Pro36, Pro37, Pro38 [Met(O)14, Asp28] Exendin-4(1-39)-NH2,
des Pro36, Pro37, Pro38 [Met(O)14, Asp28] Exendin-4(1-39)-(Lys)6-NH2,
H-(Lys)6-des Pro36, Pro37, Pro38 [Met(O)14, Asp28] Exendin-4(1-39)-(Lys)6-NH2,
H-Asn-(Glu)5 des Pro36, Pro37, Pro38 [Met(O)14, Asp28] Exendin-4(1-39)-(Lys)6-NH2,
H-Lys6-des Pro36 [Met(O)14, Trp(O2)25, Asp28] Exendin-4(1-39)-Lys6-NH2,
H-des Asp28 Pro36, Pro37, Pro38 [Met(O)14, Trp(O2)25] Exendin-4(1-39)-NH2,
H-(Lys)6-des Pro36, Pro37, Pro38 [Met(O)14, Asp28] Exendin-4(1-39)-NH2,
H-Asn-(Glu)5-des Pro36, Pro37, Pro38 [Met(O)14, Trp(O2)25, Asp28] Exendin-4(1-39)-NH2,
des Pro36, Pro37, Pro38 [Met(O)14, Trp(O2)25, Asp28] Exendin-4(1-39)-(Lys)6-NH2,
H-(Lys)6-des Pro36, Pro37, Pro38 [Met(O)14, Trp(O2)25, Asp28] Exendin-4(S1-39)-(Lys)6-NH2,
H-Asn-(Glu)5-des Pro36, Pro37, Pro38 [Met(O)14, Trp(O2)25, Asp28] Exendin-4(1-39)-(Lys)6-NH2;
or a pharmaceutically acceptable salt or solvate of any one of the afore-mentioned Exendin-4 derivative.

Hormones are for example hypophysis hormones or hypothalamus hormones or regulatory active peptides and their antagonists as listed in Rote Liste, ed. 2008, Chapter 50, such as Gonadotropine (Follitropin, Lutropin, Choriongonadotropin, Menotropin), Somatropine (Somatropin), Desmopressin, Terlipressin, Gonadorelin, Triptorelin, Leuprorelin, Buserelin, Nafarelin, Goserelin.

A polysaccharide is for example a glucosaminoglycane, a hyaluronic acid, a heparin, a low molecular weight heparin or an ultra low molecular weight heparin or a derivative thereof, or a sulphated, e.g. a poly-sulphated form of the above-mentioned polysaccharides, and/or a pharmaceutically acceptable salt thereof. An example of a pharmaceutically acceptable salt of a poly-sulphated low molecular weight heparin is enoxaparin sodium.

Antibodies are globular plasma proteins (∼150 kDa) that are also known as immunoglobulins which share a basic structure. As they have sugar chains added to amino acid residues, they are glycoproteins. The basic functional unit of each antibody is an immunoglobulin (Ig) monomer (containing only one Ig unit); secreted antibodies can also be dimeric with two Ig units as with IgA, tetrameric with four Ig units like teleost fish IgM, or pentameric with five Ig units, like mammalian IgM.

The Ig monomer is a "Y"-shaped molecule that consists of four polypeptide chains; two identical heavy chains and two identical light chains connected by disulfide bonds between cysteine residues. Each heavy chain is about 440 amino acids long; each light chain is about 220 amino acids long. Heavy and light chains each contain intrachain disulfide bonds which stabilize their folding. Each chain is composed of structural domains called Ig domains. These domains contain about 70-110 amino acids and are classified into different categories (for example, variable or V, and constant or C) according to their size and function. They have a characteristic immunoglobulin fold in which two β sheets create a "sandwich" shape, held together by interactions between conserved cysteines and other charged amino acids.

There are five types of mammalian Ig heavy chain denoted by α, δ, ε, γ, and µ. The type of heavy chain present defines the isotype of antibody; these chains are found in IgA, IgD, IgE, IgG, and IgM antibodies, respectively.

Distinct heavy chains differ in size and composition; α and γ contain approximately 450 amino acids and δ approximately 500 amino acids, while µ and ε have approximately 550 amino acids. Each heavy chain has two regions, the constant region (C_{H}) and the variable region (V_{H}). In one species, the constant region is essentially identical in all antibodies of the same isotype, but differs in antibodies of different isotypes. Heavy chains γ, α and δ have a constant region composed of three tandem Ig domains, and a hinge region for added flexibility; heavy chains µ and ε have a constant region composed of four immunoglobulin domains. The variable region of the heavy chain differs in antibodies produced by different B cells, but is the same for all antibodies produced by a single B cell or B cell clone. The variable region of each heavy chain is approximately 110 amino acids long and is composed of a single Ig domain.

In mammals, there are two types of immunoglobulin light chain denoted by λ and κ. A light chain has two successive domains: one constant domain (CL) and one variable domain (VL). The approximate length of a light chain is 211 to 217 amino acids. Each antibody contains two light chains that are always identical; only one type of light chain, κ or λ, is present per antibody in mammals.

Although the general structure of all antibodies is very similar, the unique property of a given antibody is determined by the variable (V) regions, as detailed above. More specifically, variable loops, three each the light (VL) and three on the heavy (VH) chain, are responsible for binding to the antigen, i.e. for its antigen specificity. These loops are referred to as the Complementarity Determining Regions (CDRs). Because CDRs from both VH and VL domains contribute to the antigen-binding site, it is the combination of the heavy and the light chains, and not either alone, that determines the final antigen specificity.

An "antibody fragment" contains at least one antigen binding fragment as defined above, and exhibits essentially the same function and specificity as the complete antibody of which the fragment is derived from. Limited proteolytic digestion with papain cleaves the Ig prototype into three fragments. Two identical amino terminal fragments, each containing one entire L chain and about half an H chain, are the antigen binding fragments (Fab). The third fragment, similar in size but containing the carboxyl terminal half of both heavy chains with their interchain disulfide bond, is the crystalizable fragment (Fc). The Fc contains carbohydrates, complement-binding, and FcR-binding sites. Limited pepsin digestion yields a single F(ab')2 fragment containing both Fab pieces and the hinge region, including the H-H interchain disulfide bond. F(ab')2 is divalent for antigen binding. The disulfide bond of F(ab')2 may be cleaved in order to obtain Fab'. Moreover, the variable regions of the heavy and light chains can be fused together to form a single chain variable fragment (scFv).

Pharmaceutically acceptable salts are for example acid addition salts and basic salts. Acid addition salts are e.g. HCI or HBr salts. Basic salts are e.g. salts having a cation selected from alkali or alkaline, e.g. Na+, or K+, or Ca2+, or an ammonium ion N+(R1)(R2)(R3)(R4), wherein R1 to R4 independently of each other mean: hydrogen, an optionally substituted C1-C6-alkyl group, an optionally substituted C2-C6-alkenyl group, an optionally substituted C6-C10-aryl group, or an optionally substituted C6-C10-heteroaryl group. Further examples of pharmaceutically acceptable salts are described in "Remington's Pharmaceutical Sciences" 17. ed. Alfonso R. Gennaro (Ed.), Mark Publishing Company, Easton, Pa., U.S.A., 1985 and in Encyclopedia of Pharmaceutical Technology.

Pharmaceutically acceptable solvates are for example hydrates.

## Claims

1. Medicament delivery device (1), comprising:
- a piston rod (11),
- a body (2),
- a container carrier (7) for retaining a medicament container such as a syringe (8) within the body (2), wherein the carrier (7) is movable from an initial position (IP) in a proximal direction (P) relative to the body (2),
wherein at least one longitudinal rib (2.2) associated with the piston rod (11) is arranged to engage the carrier (7), wherein at least the carrier (7) comprises a resilient material adapted to be deformed by the engagement between the carrier (7) and the at least one rib (2.2),
wherein the container carrier (7) is arranged within the body (2), wherein the piston rod (11) is connected to the body (2) and wherein the at least one rib (2.2) is arranged within the body (2) in a manner to protrude in a radial inward direction,
further comprising a sleeve (6) movable within the body (2) along a longitudinal axis so as to cover or expose an injection needle (9),
wherein the rib (2.2) comprises a step (2.3) adapted to allow the carrier (7) being moved until proximally abutting the step (2.3) without being deformed or with a lower extent of deformation and adapted to deform the carrier (7) to a greater extent proximally from the step (2.3), wherein, when the sleeve (6) is pushed against an injection site, the sleeve (6) moves in the proximal direction (P) relative to the body (2) against the force of a sleeve spring (12), wherein the container carrier (7) remains in position within the body (2) as it is stopped by the step (2.3) such that the needle (9) is exposed,
wherein, when the sleeve (6) abuts the carrier (7), the needle (9) has reached its insertion depth and further movement of the sleeve (6) relative to the body (2) in the proximal direction (P) increases the force on the carrier (7) deforming it such that it overcomes the step (2.3) and starts moving relative to the body (2) out of the initial position (IP) in the proximal direction (P).

2. Medicament delivery device (1) according to claim 1, wherein the at least one rib (2.2) comprises a resilient material adapted to be deformed by the engagement between the carrier (7) and the at least one rib (2.2).

3. Medicament delivery device (1) according to one of the preceding claims, wherein the container carrier (7) comprises a transverse portion (7.1) adapted to support a flange (8.1) on a syringe barrel (8.2) of a syringe (8) such that the syringe (8) is prevented from moving in a distal direction (D) relative to the carrier (7), wherein the transverse portion (7.1) is attached to a ring shaped collar (7.2) adapted to engage the at least one rib (2.2).

4. Medicament delivery device (1) according to claim 3, wherein the collar (7.2) has a substantially circular cross section when relaxed, wherein an internal diameter of the collar (7.2) is greater than a maximum external diameter of the flange (8.1) of the syringe (8).

5. Medicament delivery device (1) according to one of the claims 3 or 4, wherein the transverse portion (7.1) comprises an inner collar (7.5) connected to first sections (7.7) of the collar (7.2) by a number of connecting ribs (7.6).

6. Medicament delivery device (1) according to claim 5, wherein the inner collar (7.5) has an internal diameter substantially corresponding to an external diameter of the syringe barrel (8.2).

7. Medicament delivery device (1) according to one of the claims 5 or 6, wherein on axial movement of the collar (7.2) relative to the inner collar (7.5) the connecting ribs (7.6) are adapted to tilt the first sections (7.7) with respect to the longitudinal axis.

8. Medicament delivery device (1) according to one of the claims 3 to 7, wherein one or more carrier ribs (7.3) are arranged on an inner surface of the collar (7.2) and protrude in a radial inward direction in a manner to engage the flange (8.1) on the syringe barrel (8.2) for restricting movement of the syringe (8) in the proximal direction (P) relative to the carrier (7).

9. Medicament delivery device (1) according to claim 8, wherein the carrier rib (7.3) comprises a proximal ramp (7.4).

10. Medicament delivery device (1) according to one of the claims 1 to 9, wherein a sleeve spring (12) is arranged between the carrier (7) and the sleeve (6) in a manner biasing the sleeve (6) in the distal direction (D) relative to the carrier (7).

## Patentansprüche

1. Medikamenten-Verabreichungsvorrichtung (1), aufweisend:
- eine Kolbenstange (11),
- einen Körper (2),
- einen Behälterträger (7) zum Halten eines Medikamentenbehälters, beispielsweise einer Spritze (8), in dem Körper (2), wobei der Träger (7) aus einer Ausgangsposition (IP) in eine proximale Richtung (P) relativ zu dem Körper (2) beweglich ist,
wobei zumindest eine der Kolbenstange (11) zugeordnete Längsrippe (2.2) dazu angeordnet ist, den Träger (7) in Eingriff zu nehmen, wobei zumindest der Träger (7) ein federndes Material aufweist, das dazu ausgeführt ist, durch den Eingriff zwischen dem Träger (7) und der zumindest einen Rippe (2.2) deformiert zu werden,
wobei der Behälterträger (7) in dem Körper (2) angeordnet ist, wobei die Kolbenstange (11) mit dem Körper (2) verbunden ist und wobei die zumindest eine Rippe (2.2) so in dem Körper (2) angeordnet ist, dass sie in eine radial nach innen gehende Richtung vorragt,
ferner aufweisend eine Hülse (6), die in dem Körper (2) entlang einer Längsachse beweglich ist, um eine Injektionsnadel (9) abzudecken oder freizulegen,
wobei die Rippe (2.2) eine Stufe (2.3) aufweist, die dazu ausgeführt ist, zu gestatten, dass der Träger (7) bewegt wird, bis er proximal an der Stufe (2.3) anliegt, ohne deformiert zu werden oder mit einem geringeren Ausmaß an Deformation, sowie dazu ausgeführt ist, den Träger (7) proximal von der Stufe (2.3) in einem größeren Ausmaß zu deformieren, wobei sich die Hülse (6) gegen die Kraft einer Hülsenfeder (12) in die proximale Richtung (P) relativ zu dem Körper (2) bewegt, wenn die Hülse (6) gegen einen Injektionsort gedrückt wird, wobei der Behälterträger (7) in Position in dem Körper (2) bleibt, wenn er von der Stufe (2.3) gestoppt wird, so dass die Nadel (9) freigelegt wird,
wobei die Nadel (9), wenn die Hülse (6) an dem Träger (7) anliegt, ihre Einführtiefe erreicht hat und eine weitere Bewegung der Hülse (6) relativ zu dem Körper (2) in die proximale Richtung (P) die Kraft auf dem Träger (7) erhöht und ihn so deformiert, dass er die Stufe (2.3) überwindet und beginnt, sich relativ zu dem Körper (2) aus der Ausgangsposition (IP) heraus in die proximale Richtung (P) zu bewegen.

2. Medikamenten-Verabreichungsvorrichtung (1) nach Anspruch 1, wobei die zumindest eine Rippe (2.2) ein federndes Material aufweist, das dazu ausgeführt ist, durch den Eingriff zwischen dem Träger (7) und der zumindest einen Rippe (2.2) deformiert zu werden.

3. Medikamenten-Verabreichungsvorrichtung (1) nach einem der vorhergehenden Ansprüche, wobei der Behälterträger (7) einen Querabschnitt (7.1) aufweist, der dazu ausgeführt ist, einen Flansch (8.1) an einem Spritzenzylinder (8.2) einer Spritze (8) zu stützen, so dass die Spritze (8) daran gehindert wird, sich in eine distale Richtung (D) relativ zu dem Träger (7) zu bewegen, wobei der Querabschnitt (7.1) an einem ringförmigen Bund (7.2) angebracht ist, der dazu ausgeführt ist, die zumindest eine Rippe (2.2) in Eingriff zu nehmen.

4. Medikamenten-Verabreichungsvorrichtung (1) nach Anspruch 3, wobei der Bund (7.2) im entspannten Zustand einen im Wesentlichen kreisförmigen Querschnitt hat, wobei ein innerer Durchmesser des Bunds (7.2) größer als ein maximaler äußerer Durchmesser des Flanschs (8.1) der Spritze (8) ist.

5. Medikamenten-Verabreichungsvorrichtung (1) nach einem der Ansprüche 3 oder 4, wobei der Querabschnitt (7.1) einen inneren Bund (7.5) aufweist, der über mehrere Verbindungsrippen (7.6) mit ersten Sektionen (7.7) des Bunds (7.2) verbunden ist.

6. Medikamenten-Verabreichungsvorrichtung (1) nach Anspruch 5, wobei der innere Bund (7.5) einen inneren Durchmesser hat, der einem äußeren Durchmesser des Spritzenzylinders (8.2) im Wesentlichen entspricht.

7. Medikamenten-Verabreichungsvorrichtung (1) nach einem der Ansprüche 5 oder 6, wobei die Verbindungsrippen (7.6) dazu ausgeführt sind, bei axialer Bewegung des Bunds (7.2) relativ zu dem inneren Bund (7.5) die ersten Sektionen (7.7) bezüglich der Längsachse zu kippen.

8. Medikamenten-Verabreichungsvorrichtung (1) nach einem der Ansprüche 3 bis 7, wobei eine oder mehrere Trägerrippen (7.3) an einer Innenfläche des Bunds (7.2) angeordnet sind und in eine radial nach innen gehende Richtung so vorragen, dass sie den Flansch (8.1) an dem Spritzenzylinder (8.2) in Eingriff nehmen, um die Bewegung der Spritze (8) in die proximale Richtung (P) relativ zu dem Träger (7) zu beschränken.

9. Medikamenten-Verabreichungsvorrichtung (1) nach Anspruch 8, wobei die Trägerrippe (7.3) eine proximale Rampe (7.4) aufweist.

10. Medikamenten-Verabreichungsvorrichtung (1) nach einem der Ansprüche 1 bis 9, wobei eine Hülsenfeder (12) so zwischen dem Träger (7) und der Hülse (6) angeordnet ist, dass die Hülse (6) in die distale Richtung (D) relativ zu dem Träger (7) vorgespannt wird.

## Revendications

1. Dispositif d'administration de médicament (1), comprenant :
- une tige de piston (11),
- un corps (2),
- un support de récipient (7) prévu pour retenir un récipient de médicament tel qu'une seringue (8) à l'intérieur du corps (2), le support (7) pouvant être déplacé d'une position initiale (IP) dans une direction proximale (P) par rapport au corps (2),
au moins une nervure longitudinale (2.2) associée à la tige de piston (11) étant prévue pour venir en prise avec le support (7), au moins le support (7) comprenant un matériau élastique apte à être déformé par l'engagement entre le support (7) et l'au moins une nervure (2.2),
le support de récipient (7) étant disposé à l'intérieur du corps (2), la tige de piston (11) étant connectée au corps (2) et l'au moins une nervure (2.2) étant disposée à l'intérieur du corps (2) de manière à faire saillie dans une direction radiale vers l'intérieur,
comprenant en outre un manchon (6) déplaçable à l'intérieur du corps (2) le long d'un axe longitudinal de manière à couvrir ou exposer une aiguille d'injection (9),
la nervure (2.2) comprenant un gradin (2.3) apte à permettre au support (7) d'être déplacé jusqu'à ce qu'il vienne en butée proximale contre le gradin (2.3) sans être déformé ou avec une faible déformation et apte à déformer le support (7) dans une plus grande mesure dans la direction proximale depuis le gradin (2.3),
le manchon (6), lorsque le manchon (6) est poussé contre un site d'injection, se déplaçant dans la direction proximale (P) par rapport au corps (2) à l'encontre de la force d'un ressort de manchon (12), le support de récipient (7) restant en position à l'intérieur du corps (2) alors qu'il est bloqué par le gradin (2.3), de telle sorte que l'aiguille (9) soit exposée,
l'aiguille (9), lorsque le manchon (6) bute contre le support (7), ayant atteint sa profondeur d'insertion et un mouvement supplémentaire du manchon (6) par rapport au corps (2) dans la direction proximale (P) augmentant la force sur le support (7), le déformant jusqu'à ce qu'il surmonte le gradin (2.3) et qu'il commence à se déplacer par rapport au corps (2) hors de la position initiale (IP) dans la direction proximale (P) .

2. Dispositif d'administration de médicament (1) selon la revendication 1, dans lequel l'au moins une nervure (2.2) comprend un matériau élastique apte à être déformé par l'engagement entre le support (7) et l'au moins une nervure (2.2).

3. Dispositif d'administration de médicament (1) selon l'une des revendications précédentes, dans lequel le support de récipient (7) comprend une portion transversale (7.1) apte à supporter une bride (8.1) sur un cylindre de seringue (8.2) d'une seringue (8) de telle sorte que la seringue (8) ne puisse pas se déplacer dans une direction distale (D) par rapport au support (7), la portion transversale (7.1) étant attachée à un collier de forme annulaire (7.2) apte à venir en prise avec l'au moins une nervure (2.2).

4. Dispositif d'administration de médicament (1) selon la revendication 3, dans lequel le collier (7.2) présente une section transversale essentiellement circulaire lorsqu'il est relâché, un diamètre intérieur du collier (7.2) étant supérieur à un diamètre extérieur maximal de la bride (8.1) de la seringue (8).

5. Dispositif d'administration de médicament (1) selon l'une des revendications 3 et 4, dans lequel la portion transversale (7.1) comprend un collier intérieur (7.5) connecté à des premières sections (7.7) du collier (7.2) par un certain nombre de nervures de connexion (7.6).

6. Dispositif d'administration de médicament (1) selon la revendication 5, dans lequel le collier intérieur (7.5) présente un diamètre intérieur correspondant substantiellement à un diamètre extérieur du cylindre de seringue (8.2).

7. Dispositif d'administration de médicament (1) selon l'une des revendications 5 et 6, dans lequel, lors du mouvement axial du collier (7.2) par rapport au collier intérieur (7.5), les nervures de connexion (7.6) sont aptes à faire basculer les premières sections (7.7) par rapport à l'axe longitudinal.

8. Dispositif d'administration de médicament (1) selon l'une des revendications 3 à 7, dans lequel une ou plusieurs nervures de support (7.3) sont disposées sur une surface intérieure du collier (7.2) et font saillie dans une direction radiale vers l'intérieur de manière à venir en prise avec la bride (8.1) sur le cylindre de seringue (8.2) pour limiter le mouvement de la seringue (8) dans la direction proximale (P) par rapport au support (7) .

9. Dispositif d'administration de médicament (1) selon la revendication 8, dans lequel la nervure de support (7.3) comprend une rampe proximale (7.4) .

10. Dispositif d'administration de médicament (1) selon l'une des revendications 1 à 9, dans lequel un ressort de manchon (12) est disposé entre le support (7) et le manchon (6) de manière à solliciter le manchon (6) dans la direction distale (D) par rapport au support (7).
